# EUROPEAN PATENT APPLICATION

(11) **EP 1 800 666 A1**
(43) Date of publication of application: **27.06.2007**
(21) Application number: 06022201.5
(22) Date of filing: 20.03.2003
(51) Int. Cl.: A61K 9/14, A61K 31/565

(54) **Nanoparticulate compositions of angiogenesis inhibitors**

(30) Priority: 20.03.2002 US 365540 P; 25.03.2002 US 366542 P
(62) Divisional of application: 03723781.5
(71) Applicant: Elan Pharma International Limited, Shannon, County Clare (IE)
(72) Inventor: Merisko-Liversidge, Elaine, West Chester, PA 19380 (US); Bosch, H.William, Bryn Mawr, PA 19010 (US); Cary, Greta G., Landsdale, PA 19446 (US); Pruitt, John, Collegeville, PA 19426 (US); Ryde, Tuula, Malvern, PA 19355 (US); Jain, Rajeev, Farmingham, MA 10701 (US); Weiderhold, Amy, North Wales, PA 19454 (US)
(74) Representative: Wichmann, Hendrik

(57) **Abstract**

Nanoparticulate compositions comprising at least one poorly soluble angiogenesis inhibitor and at least one surface stabilizer are described. The nanoparticulate compositions have an average particle size of less than about 2000 nm. The invention also describes methods of making and using such compositions.

## Description

### FIELD OF THE INVENTION

The present invention is directed to nanoparticulate formulations of angiogenesis inhibitors and methods of making and using such compositions.

### BACKGROUND OF THE INVENTION

### A. Background Regarding Nanoparticulate Compositions

Nanoparticulate compositions, first described in U.S. Patent No. 5,145,684 ("the '684 patent"), are particles consisting of a poorly soluble therapeutic or diagnostic agent having adsorbed onto the surface thereof a non-crosslinked surface stabilizer. This invention is an improvement over that disclosed in the '684 patent, as the '684 patent does not describe nanoparticulate compositions comprising an angiogenesis inhibitor.

The '684 patent describes a method of screening active agents to identify useful surface stabilizers that enable the production of a nanoparticulate composition. Not all surface stabilizers will function to produce a stable, non-agglomerated nanoparticulate composition for all active agents.

Methods of making nanoparticulate compositions are described in, for example, U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510, 118 for "Process of Preparing Therapeutic Compositions Containing Nanoparticles."

Nanoparticulate compositions are also described in, for example, U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate," 6,428,814 for "Bioadhesive nanoparticulate compositions having cationic surface stabilizers;" 6,431,478 for "Small Scale Mill;" and 6,432,381 for "Methods for targeting drug delivery to the upper and/or lower gastrointestinal tract," all of which are specifically incorporated by reference. In addition, U.S. Patent Application No. 20020012675 A1, published on January 31, 2002, for "Controlled Release Nanoparticulate Compositions," describes nanoparticulate compositions, and is specifically incorporated by reference.

Amorphous small particle compositions are described in, for example, U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter."

### B. Background Regarding Angiogenesis Inhibitors

Angiogenesis means the formation of new blood vessels. Tumor angiogenesis is the growth of blood vessels from surrounding tissue to a solid tumor, caused by the release of chemicals by the tumor. Other chemicals, called angiogenesis inhibitors, signal the process to stop. Angiogenesis plays an important role in the growth and spread of cancer, as new blood vessels "feed" the cancer cells with oxygen and nutrients, allowing these cells to grow, invade nearby tissue, spread to other parts of the body, and form new colonies of cancer cells. Because cancer cannot grow or spread without the formation of new blood vessels, angiogenesis inhibitors can be useful in preventing the growth of cancer by blocking the formation of new blood vessels from surrounding tissue to a solid tumor. This in turn might stop the tumor from growing and spreading to other parts of the body. In animal studies, angiogenesis inhibitors have successfully stopped the formation of new blood vessels, causing the cancer to shrink and die. *See* http://cis.nci.nih.gov/fact/742.htm.

Exemplary angiogenesis inhibitors given at cancer.gov (affiliated with the National Institutes of Health) are provided in the following table.

| **Agent** | **Description** |
|---|---|
| 2-methoxyestradiol | 2ME. A drug derived from estrogen that belongs to the family of drugs called angiogenesis inhibitors. It prevents the formation of new blood vessels that tumors need to grow. |
| AG3340 | An anticancer drug that belongs to the family of drugs called angiogenesis inhibitors. AG3340 is a matrix metalloproteinase (MMP) inhibitor. Also called prinomastat. |
| batimastat | An anticancer drug that belongs to the family of drugs called angiogenesis inhibitors. Batimastat is a matrix metalloproteinase inhibitor. |
| BAY 12-9566 | An anticancer drug that belongs to the family of drugs called angiogenesis inhibitors. |
| carboxyamidotriazole | An anticancer drug that belongs to the family of drugs called angiogenesis inhibitors. |
| CC-1088 | A drug that is similar but not identical to thalidomide and is being studied as an anticancer drug. It belongs to the family of drugs called angiogenesis inhibitors. |
| dextromethorphan acetic acid | An anticancer drug that belongs to the family of drugs called angiogenesis inhibitors. |
| dimethylxanthenone acetic acid | An anticancer drug that belongs to the family of drugs called angiogenesis inhibitors. |
| EMD 121974 | A substance that is being studied as an anticancer and antiangiogenesis drug. |
| endostatin | A drug that is being studied for its ability to prevent the growth of new blood vessels into a solid tumor. Endostatin belongs to the family of drugs called angiogenesis inhibitors. |
| IM-862 | An anticancer drug that belongs to the family of drugs called angiogenesis inhibitors. |
| marimastat | An anticancer drug that belongs to the family of drugs called angiogenesis inhibitors. Marimastat is a MMP inhibitor. |
| matrix metalloproteinase | A member of a group of enzymes that can break down proteins, such as collagen, that are normally found in the spaces between cells in tissues (i.e., extracellular matrix proteins). Because these enzymes need zinc or calcium atoms to work properly, they are called metalloproteinases. Matrix metalloproteinases are involved in wound healing, angiogenesis, and tumor cell metastasis. |
| penicillamine | A drug that removes copper from the body arid is used to treat diseases in which there is an excess of this metal. It is also being studied as a possible angiogenesis inhibitor in brain tumors. |
| PTK787/ZK 222584 | An anticancer drug that belongs to the family of drugs called angiogenesis inhibitors. |
| RPI.4610 | A substance that is being studied as a treatment for cancer. It belongs to the family of drugs called angiogenesis inhibitors. |
| squalamine lactate | A drug that belongs to the family of drugs called angiogenesis inhibitors. It prevents the growth of new blood vessels into a solid tumor. |
| SU5416 | An anticancer drug that belongs to the family of drugs called angiogenesis inhibitors. SU5416, 3-[2, 4-dimethylpyrrol-5-yl methylidenyl]-2-indolinone, has the following structure http://www.pharmquest.com/source/features/ AAPS_Trends_eRD/SUCEN_Arun_Koparkar.pdf): |
| | 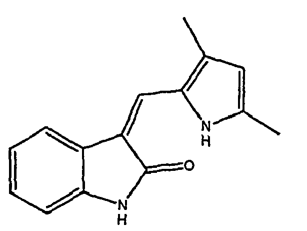 |
| thalidomide | A drug that belongs to the family of drugs called angiogenesis inhibitors. It prevents the growth of new blood vessels into a solid tumor. |
| TNP-470 | A drug that belongs to the family of drugs called angiogenesis inhibitors. It prevents the growth of new blood vessels into a solid tumor. |

Other known angiogenesis inhibitors include, but are not limited to, suramin, combretastatin, paclitaxel, and tamoxifen,. One of these compounds, suramin, is soluble in water. More detailed descriptions of select angiogenesis inhibitors are given below.

Combretastatin was disclosed in the Journal of the National Cancer Institute on April 5, 2000, as an angiogenesis inhibitor isolated from the bark of a South African species of willow tree. The compound is described and claimed in U.S. Patent No. 4,996,237, assigned to the Arizona Board of Regents.

2-methoxyestradiol was disclosed in the Journal of the National Cancer Institute on April 5, 2000, as an angiogenesis inhibitor. In a press release of February 14, 2000, Entremed, Inc., in Rockville, MD was given permission for Phase I trials of 2ME2. Entremed provides an overview of 2ME2 on their web site. Claim 2 of U.S. Patent No. 5,504,074 is directed to a method for treating mammalian disease characterized by undesirable angiogenesis comprising administering 2-methoxyestradiol.

At the 54^{th} meeting of the Department of Health and Human Services, Food and Drug Administration, Center for Drug Evaluation and Research, Division of Oncology, the director of the Angiogenesis Foundation informed the committee about the angiogenesis inhibitory activity of paclitaxel. The Merck Index listing of Taxol (trademark name of paclitaxel) states that the compound was first isolated from the bark of the Pacific yew tree.

At the 58^{th} meeting of the Department of Health and Human Services, Food and Drug Administration, Center for Drug Evaluation and Research, Oncologic Drugs Advisory Committee, it was reported that tamoxifen is an angiogenesis inhibitor. Conventional tamoxifen is generic, as its isolation and identification were described in the 1960s. However, isomers of tamoxifen are patented. *See e.g.,* claim 2 of U.S. Patent No. 4,536,516,.

Newton, "Novel Chemotherapeutic Agents for the Treatment of Brain Cancer," Expert Opin. Investigational Drugs, 9:2815-29 (2000), discloses that neoplastic angiogenesis and brain tumor invasion are also targets for therapeutic interventions with new agents such as thalidomide, suramin, and marimastat.

Liekens et al., "Angiogenesis: Regulators and Clinical Applications," Biochem. Pharmacol., 61i:253-70 (2001), disclose that TNP-470 is an angiogenesis inhibitor. Claim 1 of U.S. Patent No. 5,166,172, assigned to Takeda Chemical Industries, Ltd., is directed to O-(chloroacetylcarbamoyl) fumagillol (TNP-470). Example 8 of this patent discloses that TNP-470 is obtained from silica gel with a mixture of n-hexane and ethylacetate.

Experiments examining thalidomide's enantiomers reveal that the S(-)- enantiomer has the strongest antiangiogenic activity. Kenyon et al., "Effects of thalidomide and related metabolites in a mouse corneal model of neovascularization," Exp. Eye Res., 64:971-978 (1997). Moreover, the immunomodulating and anti-inflammatory effects of thalidomide are likely chiefly exerted by S-thalidomide. Eriksson et al., "Intravenous formulations of the enantiomers of thalidomide: Pharmacokinetic and initial pharmacodynamic characterization in man," J. Pharm. Pharmacol., 52:807-817 (2000).

Other studies have shown that the R-isomer provides the drug's sedative effect, and that the S-isomer is responsible for the birth defects associated with the agent. C. Star, "Splitting pairs: molecular maneuver aims for better drugs," Drug Topics, 136(15):26 (Aug. 3, 1992).

U.S. Patent No. 6,124,322 teaches that pure enantiomers of thalidomide are converted back into the racemate *in vitro* and *in vivo. See also Drug Topics,* above. The antipode is formed immediately after the parenteral administration of one of the isomers of thalidomide *in vivo,* and an equilibrium is established after about 4 hours.

The claims of U.S. Patent No. 6,124,322 recite aqueous thalidomide solutions of either the R or S enantiomers of thalidomide. According to the disclosure of the patent, the enantiomers are more soluble than the racemate of thalidomide, which enables intravenous administration of the enantiomers.

Angiogenesis inhibitors currently in clinical trials include the following (http://www.cancer.gov/clinical_trials/doc.aspx?viewid=B0959CBB-3004-4160-A679-6DD204BEE68C): marimastat, COL-3 (synthetic MMP inhibitor; tetracycline derivative), neovastat (naturally occurring MMP inhibitor), BMS-275291 (synthetic MMP inihibitor), thalidomide, squalamine (extract from dogfish shark liver; inhibits sodium-hydrogen exchanger, NHE3), 2-ME (inhibition of endothelial cells), SU6668 (blocks VEGF, FGF, and PDGF receptor signaling), interferon-alpha (inhibition of bFGF and VEGF production), anti-VEGF antibody (monoclonal antibody to vascular endothelial growth factor (VEGF)), Medi-522 (Vitaxin II) (antibody that blocks the integrin present on endothelial cell surface), EMD121974 (small molecule blocker of integrin present on endothelial cell surface), CAI (inhibitor of calcium influx), celecoxib (enzyme cyclooxygenase 2 (COX-2)), Interleukin-12 (up-regulation of interferon gamma and IP-10), and IM862 (unknown mechanism).

Additionally, the following angiogenesis inhibitors are disclosed in the CalBioChem® catalog at page xxxiii: Amilloride, Human Angiostatin® Protein, Human Angiostatin K1-3, Human Angiostatin K1-5, Captopril, DL-alpha-Difluoromethylornithine HCl, Human Recombinant Endostatin^{™} Protein (*Pichia pastoris),* Mouse Recombinant Endostatin^{™} Protein (*Pichia pastoris),* Mouse Recombinant His-Tag® Endostatin^{™} Protein *(Spodoptera* frugiperda), Fumagillin *(Aspergillus fumagatus),* Herbimycin A *(Streptomyces* sp), 4-Hydroxyphenylretinamide, Mouse Recombinant alpha-interferon *(E. coli),* Human Recombinant gamma-interferon *(E. coli),* Juglone, Laminin Hexapeptide, Laminin Pentapeptide, Lavendustin A, Medroxyprogesterone Acetate, 2-Methoxyestradiol, Minocycline HCl, Human Recombinant Placental Ribonuclease Inhibitor, Sodium Salt Suramin, (±)-Thalidomide Human Platelet Thrombospondin, Recombinant Bovine Tissue Inhibitor of Metalloproteinase 1, Recombinant Human Tissue Inhibitor of Metalloproteinase 1, Recombinant Human Neutrophil Granulocyte Tissue Inhibitor of Metalloproteinase 1, and Recombinant Human Rheumatoid Synovial Fibroblast Tissue Inhibitor of Metalloproteinase 2.

There is a need in the art for nanoparticulate compositions of angiogenesis inhibitors and methods of making and using such compositions. The present invention satisfies these needs.

### SUMMARY OF THE INVENTION

The present invention is directed to nanoparticulate compositions comprising at least one poorly soluble angiogenesis inhibitor and at least one surface stabilizer associated with the surface of the angiogenesis inhibitor.

Another aspect of the invention is directed to pharmaceutical compositions comprising a nanoparticulate angiogenesis inhibitor composition of the invention. The pharmaceutical compositions preferably comprise at least one poorly soluble angiogenesis inhibitor, at least one surface stabilizer associated with the surface of the inhibitor, and a pharmaceutically acceptable carrier, as well as any desired excipients.

This invention further discloses a method of making a nanoparticulate composition having at least one poorly soluble angiogenesis inhibitor and at least one surface stabilizer associated with the surface of the inhibitor. Such a method comprises contacting a poorly soluble nanoparticulate angiogenesis inhibitor with at least one surface stabilizer for a time and under conditions sufficient to provide an angiogenesis inhibitor/surface stabilizer composition. The surface stabilizer can be contacted with the angiogenesis inhibitor either before, during, or after particle size reduction of the angiogenesis inhibitor.

The present invention is further directed to a method of treatment comprising administering to a mammal a therapeutically effective amount of a nanoparticulate angiogenesis inhibitor composition according to the invention.

Both the foregoing general description and the following detailed description are exemplary and explanatory and are intended to provide further explanation of the invention as claimed. Other objects, advantages, and novel features will be readily apparent to those skilled in the art from the following detailed description of the invention.

### DETAILED DESCRIPTION OF THE INVENTION

The present invention is directed to the surprising and unexpected discovery that stable nanoparticulate compositions of angiogenesis inhibitors can be made.

Advantages of the angiogenesis inhibitor compositions of the invention include, but are not limited to: (1) faster onset of action; (2) smaller tablet or other solid dosage form size, or smaller volume if in a liquid dosage form; (3) smaller doses of drug required to obtain the same pharmacological effect as compared to conventional microcrystalline forms of the same angiogenesis inhibitor; (4) increased bioavailability as compared to conventional microcrystalline forms of the same angiogenesis inhibitor; (5) substantially similar pharmacokinetic profiles of the angiogenesis inhibitor compositions of the invention when administered in the fed versus the fasted state; (6) bioequivalency of the angiogenesis inhibitor compositions of the invention when administered in the fed versus the fasted state; (7) improved pharmacokinetic profiles; (8) an increased rate of dissolution for the angiogenesis inhibitor compositions of the invention as compared to conventional microcrystalline forms of the same angiogenesis inhibitor; (9) bioadhesive angiogenesis inhibitor compositions; (10) the angiogenesis inhibitor compositions of the invention can be sterile filtered; and (11) the angiogenesis inhibitor compositions of the invention can be used in conjunction with other active agents.

The invention encompasses the angiogenesis inhibitor compositions of the invention formulated or coadministered with one or more non-angiogenesis inhibitor active agents, either conventional (solubilized or microparticulate) or nanoparticulate. Methods of using such combination compositions are also encompassed by the invention.

The present invention is described herein using several definitions, as set forth below and throughout the application.

"About" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

As used herein with reference to stable drug particles, 'stable' means that angiogenesis inhibitor particles do not appreciably flocculate or agglomerate due to interparticle attractive forces or otherwise increase in particle size.

"Therapeutically effective amount" as used herein with respect to a drug dosage, shall mean that dosage that provides the specific pharmacological response for which the drug is administered in a significant number of subjects in need of such treatment. It is emphasized that 'therapeutically effective amount,' administered to a particular subject in a particular instance will not always be effective in treating the diseases described herein, even though such dosage is deemed a 'therapeutically effective amount' by those skilled in the art. It is to be further understood that drug dosages are, in particular instances, measured as oral dosages, or with reference to drug levels as measured in blood.

"Conventional active agents or drugs" refers to non-nanoparticulate or solubilized active agents or drugs. Non-nanoparticulate active agents have an effective average particle size of greater than about 2 microns.

### A. Preferred Characteristics of the Angiogenesis Inhibitor Compositions of the Invention

### 1. Fast Onset of Activity

The use of conventional formulations of angiogenesis inhibitors is not ideal due to delayed onset of action. In contrast, the nanoparticulate angiogenesis inhibitor compositions of the invention exhibit faster therapeutic effects. Moreover, nanoparticulate formulations of angiogenesis inhibitors enable selection of an angiogenesis inhibitor with a long half-life in the blood stream while still providing the subject with a fast-acting compound.

Preferably, following administration the angiogenesis inhibitor compositions of the invention have a Tₘₐₓ of less than about 2.5 hours, less than about 2.25 hours, less than about 2 hours, less than about 1.75 hours, less than about 1.5 hours, less than about 1.25 hours, less than about 1.0 hours, less than about 50 minutes, less than about 40 minutes, less than about 30 minutes, less than about 25 minutes, less than about 20 minutes, less than about 15 minutes, or less than about 10 minutes.

### 2. Increased Bioavailability

The angiogenesis inhibitor compositions of the invention preferably exhibit increased bioavailability, at the same dose of the same angiogenesis inhibitor, and require smaller doses, as compared to prior conventional angiogenesis inhibitor compositions.

Any drug, including angiogenesis inhibitors, can have adverse side effects. Thus, lower doses of angiogenesis inhibitors which can achieve the same or better therapeutic effects as those observed with larger doses of conventional angiogenesis inhibitors are desired. Such lower doses can be realized with the angiogenesis inhibitor compositions of the invention, because the greater bioavailability observed with the nanoparticulate angiogenesis inhibitor compositions as compared to conventional drug formulations means that smaller does of drug are required to obtain the desired therapeutic effect.

### 3. The Pharmacokinetic Profiles of the Angiogenesis Inhibitor Compositions of the Invention are not Substantially Affected by the Fed or Fasted State of the Subject Ingesting the Compositions

The invention encompasses an angiogenesis inhibitor composition wherein the pharmacokinetic profile of the angiogenesis inhibitor is not substantially affected by the fed or fasted state of a subject ingesting the composition. This means that there is no substantial difference in the quantity of drug absorbed or the rate of drug absorption when the nanoparticulate angiogenesis inhibitor compositions are administered in the fed versus the fasted state. Thus, the nanoparticulate angiogenesis inhibitor compositions of the invention substantially eliminate the effect of food on the pharmacokinetics of the angiogenesis inhibitor.

Preferably, the difference in absorption of the nanoparticulate angiogenesis inhibitor compositions of the invention, when administered in the fed versus the fasted state, is less than about 100%, less than about 90%, less than about 80%, less than about 70%, less than about 60%, less than about 50%, less than about 40%, less than about 35%, less than about 30%, less than about 25%, less than about 20%, less than about 15%, less than about 10%, less than about 5%, less than about 3%, or essentially no difference.

In addition, preferably the difference in the rate of absorption (i.e., Tₘₐₓ) of the nanoparticulate angiogenesis inhibitor compositions of the invention, when administered in the fed versus the fasted state, is less than about 100%, less than about 90%, less than about 80%, less than about 70%, less than about 60%, less than about 50%, less than about 40%, less than about 30%, less than about 20%, less than about 15%, less than about 10%, less than about 5%, less than about 3%, or essentially no difference.

Benefits of a dosage form which substantially eliminates the effect of food include an increase in subject convenience, thereby increasing subject compliance, as the subject does not need to ensure that they are taking a dose either with or without food.

### 4. Redispersibility Profiles of the Angiogenesis Inhibitor Compositions of the Invention

An additional feature of the angiogenesis inhibitor compositions of the invention is that the compositions redisperse such that the effective average particle size of the redispersed angiogenesis inhibitor particles is less than about 2 microns. This is significant, as if upon administration the nanoparticulate angiogenesis inhibitor compositions of the invention did not redisperse to a substantially nanoparticulate particle size, then the dosage form may lose the benefits afforded by formulating the angiogenesis inhibitor into a nanoparticulate particle size.

This is because nanoparticulate angiogenesis inhibitor compositions benefit from the small particle size of the angiogenesis inhibitor; if the nanoparticulate angiogenesis inhibitor particles do not redisperse into the small particle sizes upon administration, then "clumps" or agglomerated angiogenesis inhibitor particles are formed, owing to the extremely high surface free energy of the nanoparticulate system and the thermodynamic driving force to achieve an overall reduction in free energy. With the formation of such agglomerated particles, the bioavailability of the dosage form may fall well below that observed with the liquid dispersion form of the nanoparticulate angiogenesis inhibitor composition.

Preferably, the redispersed angiogenesis inhibitor particles of the invention have an effective average particle size of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

### 5. Bioadhesive Angiogenesis Inhibitor Compositions

Bioadhesive angiogenesis inhibitor compositions of the invention comprise at least one cationic surface stabilizer, which are described in more detail below. Bioadhesive formulations of angiogenesis inhibitors exhibit exceptional bioadhesion to biological surfaces, such as mucous. The term bioadhesion refers to any attractive interaction between two biological surfaces or between a biological and a synthetic surface. In the case of bioadhesive nanoparticulate angiogenesis inhibitor compositions, the term bioadhesion is used to describe the adhesion between the nanoparticulate angiogenesis inhibitor compositions and a biological substrate (i.e. gastrointestinal mucin, lung tissue, nasal mucosa, *etc.*)*. See e.g.,* U.S. Patent No. 6,428,814 for "Bioadhesive Nanoparticulate Compositions Having Cationic Surface Stabilizers," which is specifically incorporated by reference.

The bioadhesive angiogenesis inhibitor compositions of the invention are useful in any situation in which it is desirable to apply the compositions to a biological surface. The bioadhesive angiogenesis inhibitor compositions coat the targeted surface in a continuous and uniform film which is invisible to the naked human eye.

A bioadhesive angiogenesis inhibitor composition slows the transit of the composition, and some angiogenesis inhibitor particles would also most likely adhere to tissue other than the mucous cells and therefore give a prolonged exposure to the angiogenesis inhibitor, thereby increasing absorption and the bioavailability of the administered dosage.

### 6. Pharmacokinetic Profiles of the Angiogenesis Inhibitor Compositions of the Invention

The present invention provides compositions of one or more angiogenesis inhibitors having a desirable pharmacokinetic profile when administered to mammalian subjects. Preferably, the Tₘₐₓ of an administered dose of a nanoparticulate angiogenesis inhibitor is less than that of a conventional non-nanoparticulate composition of the same angiogenesis inhibitor, administered at the same dosage. In addition, preferably the Cₘₐₓ of a nanoparticulate composition of an angiogenesis inhibitor is greater than the Cₘₐₓ of a conventional non-nanoparticulate composition of the same angiogenesis inhibitor, administered at the same dosage.

In comparative pharmacokinetic testing with a non-nanoparticulate composition of an angiogenesis inhibitor, a nanoparticulate composition of the same angiogenesis inhibitor, administered at the same dosage, preferably exhibits a Tₘₐₓ which is less than about 100%, less than about 90%, less than about 80%, less than about 70%, less than about 60%, less than about 50%, less than about 40%, less than about 30%, less than about 25%, less than about 20%, less than about 15%, or less than about 10% of the Tₘₐₓ exhibited by the non-nanoparticulate composition of the angiogenesis inhibitor.

In comparative pharmacokinetic testing with a non-nanoparticulate composition of an angiogenesis inhibitor, a nanoparticulate composition of the same angiogenesis inhibitor, administered at the same dosage, preferably exhibits a Cₘₐₓ which is greater than about 5%, greater than about 10%, greater than about 15%, greater than about 20%, greater than about 30%, greater than about 40%, greater than about 50%, greater than about 60%, greater than about 70%, greater than about 80%, greater than about 90%, greater than about 100%, greater than about 110%, greater than about 120%, greater than about 130%, greater than about 140%, or greater than about 150% than the Cₘₐₓ exhibited by the non-nanoparticulate composition of the angiogenesis inhibitor.

The desirable pharmacokinetic profile, as used herein, is the pharmacokinetic profile measured after an initial dose of an angiogenesis inhibitor. The compositions can be formulated in any way as described below.

### C. Combination Pharmacokinetic Profile Compositions

In yet another embodiment of the invention, a first angiogenesis inhibitor composition providing a desired pharmacokinetic profile is co-administered, sequentially administered, or combined with at least one other angiogenesis inhibitor composition that generates a desired different pharmacokinetic profile. More than two angiogenesis inhibitor compositions can be co-administered, sequentially administered, or combined. While at least one of the angiogenesis inhibitor compositions has a nanoparticulate particle size, the additional one or more angiogenesis inhibitor compositions can be nanoparticulate, solubilized, or have a conventional microparticulate particle size.

For example, a first angiogenesis inhibitor composition can have a nanoparticulate particle size, conferring a short Tₘₐₓ and typically a higher Cₘₐₓ. This first angiogenesis inhibitor composition can be combined, co-administered, or sequentially administered with a second composition comprising: (1) a different nanoparticulate angiogenesis inhibitor exhibiting slower absorption and, therefore a longer Tₘₐₓ and typically a lower Cₘₐₓ; (2) the same angiogenesis inhibitor having a larger (but still nanoparticulate) particle size, and therefore exhibiting slower absorption, a longer Tₘₐₓ, and typically a lower Cₘₐₓ; or (3) a microparticulate angiogenesis inhibitor composition (with the angiogenesis inhibitor being either the same as or different from the angiogenesis inhibitor of the first composition), exhibiting a longer Tₘₐₓ, and typically a lower Cₘₐₓ.

The second, third, fourth, *etc.,* angiogenesis inhibitor composition can differ from the first, and from each other, for example: (1) in the identity of the angiogenesis inhibitor; (2) in the effective average particle sizes of each composition; or (3) in the dosage of the angiogenesis inhibitor. Angiogenesis inhibitor compositions can produce a different Tₘₐₓ. Such a combination composition can reduce the dose frequency required.

If the second angiogenesis inhibitor composition has a nanoparticulate particle size, then preferably the angiogenesis inhibitor has at least one surface stabilizer associated with the surface of the drug particles. The one or more surface stabilizers can be the same as or different from the surface stabilizers associated with the surface of the first angiogenesis inhibitor.

Preferably where co-administration of a "fast-acting" formulation and a "longer-lasting" formulation is desired, the two formulations are combined within a single composition, for example a dual-release composition.

### D. Compositions

The compositions of the invention comprise at least one poorly soluble angiogenesis inhibitor and at least one surface stabilizer. Surface stabilizers useful herein associate with the surface of the nanoparticulate angiogenesis inhibitor, but do not chemically react with the angiogenesis inhibitor or itself. Preferably, individually adsorbed molecules of the surface stabilizer are essentially free of intermolecular cross-linkages.

The present invention also includes nanoparticulate angiogenesis inhibitors having at least one surface stabilizer associated with the surface thereof, formulated into compositions together with one or more non-toxic physiologically acceptable carriers, adjuvants, or vehicles, collectively referred to as carriers.

### 1. Angiogenesis Inhibitor Drug Particles

The compositions of the invention comprise a poorly soluble angiogenesis inhibitor which is dispersible in at least one liquid medium. The angiogenesis inhibitor exists as a discrete crystalline phase, as an amorphous phase, a semi-crystalline phase, a semi-amorphouse phase, or a combination thereof. The crystalline phase differs from a non-crystalline or amorphous phase which results from precipitation techniques, such as those described in EP Patent No. 275,796. By "poorly soluble" it is meant that the angiogenesis inhibitor has a solubility in a liquid dispersion medium of less than about 30 mg/mL, less than about 20 mg/mL, less than about 10 mg/mL, or less than about 1 mg/mL. Useful liquid dispersion mediums include, but are not limited to, water, aqueous salt solutions, safflower oil, and solvents such as ethanol, t-butanol, hexane, and glycol.

Useful angiogenesis inhibitors according to the invention include, but are not limited to: 2-methoxyestradiol, prinomastat, batimastat, BAY 12-9566, carboxyamidotriazole, CC-1088, dextromethorphan acetic, dimethylxanthenone acetic acid, EMD 121974, endostatin, IM-862, marimastat, matrix metalloproteinase, penicillamine, PTK787/ZK 222584, RPI.4610, squalamine, squalamine lactate, SU5416, (±)-thalidomide, S- thalidomide, R- thalidomide, TNP-470, combretastatin, paclitaxel, tamoxifen, COL-3, neovastat, BMS-275291, SU6668, interferon-alpha, anti-VEGF antibody, Medi-522 (Vitaxin II), CAI, celecoxib, Interleukin-12, IM862, Amilloride, Angiostatin® Protein, Angiostatin K1-3, Angiostatin K1-5, Captopril, DL-alpha-Difluoromethylomithine, DL-alpha-Difluoromethylomithine HCl, His-Tag® Endostatin^{™} Protein, Fumagillin, Herbimycin A, 4-Hydroxyphenylretinamide, gamma-interferon, Juglone, Laminin, Laminin Hexapeptide, Laminin Pentapeptide, Lavendustin A, Medroxyprogesterone, Medroxyprogesterone Acetate, Minocycline, Minocycline HCl, Placental Ribonuclease Inhibitor, Suramin, Sodium Salt Suramin, Human Platelet Thrombospondin, Tissue Inhibitor of Metalloproteinase 1, Neutrophil Granulocyte Tissue Inhibitor of Metalloproteinase 1, and Rheumatoid Synovial Fibroblast Tissue Inhibitor of Metalloproteinase 2. *See* http://cis.nci.nih.gov/fact/7 42.htm; CalBioChem® catalog at page xxxiii; and http://www.cancer.gov/clinical_trials/doc.aspx?viewid=B0959CBB-3004-4160-A679-6DD204BEE68C.

### 2. Non-Angiogenesis Inhibitor Active Agents

The nanoparticulate angiogenesis inhibitor compositions of the invention can additionally comprise one or more non-angiogenesis inhibitor active agents, in either a conventional or nanoparticulate particle size. The non-angiogenesis inhibitor active agents can be present in a crystalline phase, an amorphous phase, a semi-crystalline phase, a semi-amorphous phase, or a mixture thereof.

If the non-angiogenesis inhibitor active agent has a nanoparticulate particle size *i.e.,* a particle size of less than about 2 microns, then preferably it will have one or more surface stabilizers associated with the surface of the active agent. In addition, if the active agent has a nanoparticulate particle size, then it is preferably poorly soluble and dispersible in at least one liquid dispersion medium. By "poorly soluble" it is meant that the active agent has a solubility in a liquid dispersion medium of less than about 30 mg/mL, less than about 20 mg/mL, less than about 10 mg/mL, or less than about 1 mg/mL. Useful liquid dispersion mediums include, but are not limited to, water, aqueous salt solutions, safflower oil, and solvents such as ethanol, t-butanol, hexane, and glycol.

Such active agents can be, for example, a therapeutic agent. A therapeutic agent can be a pharmaceutical agent, including biologics such as amino acids, proteins, peptides, and nucleotides. The active agent can be selected from a variety of known classes of drugs, including, for example, amino acids, proteins, peptides, nucleotides, anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, alpha-adrenergic receptor blocking agents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immunological agents, lipid regulating agents, muscle relaxants, parasympathomimetics, parathyroid calcitonin and biphosphonates, prostaglandins, radio- pharmaceuticals, sex hormones (including steroids), anti-allergic agents, stimulants and anoretics, sympathomimetics, thyroid agents, vasodilators, and xanthines.

A description of these classes of active agents and a listing of species within each class can be found in Martindale's The Extra Pharmacopoeia, 31st Edition (The Pharmaceutical Press, London, 1996), specifically incorporated by reference. The active agents are commercially available and/or can be prepared by techniques known in the art.

Exemplary nutraceuticals and dietary supplements are disclosed, for example, in Roberts et al., Nutraceuticals: The Complete Encyclopedia of Supplements, Herbs, Vitamins, and Healing Foods (American Nutraceutical Association, 2001), which is specifically incorporated by reference. Dietary supplements and nutraceuticals are also disclosed in Physicians' Desk Reference for Nutritional Supplements, 1st Ed. (2001) and The Physicians' Desk Reference for Herbal Medicines, 1st Ed. (2001), both of which are also incorporated by reference. A nutraceutical or dietary supplement, also known as phytochemicals or functional foods, is generally any one of a class of dietary supplements, vitamins, minerals, herbs, or healing foods that have medical or pharmaceutical effects on the body.

Exemplary nutraceuticals or dietary supplements include, but are not limited to, lutein, folic acid, fatty acids (e.g., DHA and ARA), fruit and vegetable extracts, vitamin and mineral supplements, phosphatidylserine, lipoic acid, melatonin, glucosamine/chondroitin, Aloe Vera, Guggul, glutamine, amino acids (e.g., arginine, isoleucine, leucine, lysine, methionine, phenylanine, threonine, tryptophan, and valine), green tea, lycopene, whole foods, food additives, herbs, phytonutrients, antioxidants, flavonoid constituents of fruits, evening primrose oil, flax seeds, fish and marine animal oils, and probiotics. Nutraceuticals and dietary supplements also include bio-engineered foods genetically engineered to have a desired property, also known as "pharmafoods."

The compound to be administered in combination with a nanoparticulate angiogenesis inhibitor composition of the invention can be formulated separately from the angiogenesis inhibitor composition or co-formulated with the angiogenesis inhibitor composition. Where an angiogenesis inhibitor composition is co-formulated with a second active agent, the second active agent can be formulated in any suitable manner, such as immediate-release, rapid-onset, sustained-release, or dual-release form.

### 3. Surface Stabilizers

Useful surface stabilizers, which are known in the art and described in the '684 patent, are believed to include those which associate with the surface of the angiogenesis inhibitor but do not chemically bond to or interact with the angiogenesis inhibitor. The surface stabilizer is associated with the surface of the angiogenesis inhibitor in an amount sufficient to maintain the angiogenesis inhibitor particles at an effective average particle size of less than about 2000 nm. Furthermore, the individually adsorbed molecules of the surface stabilizer are preferably essentially free of intermolecular cross-linkages. Two or more surface stabilizers can be employed in the compositions and methods of the invention.

Suitable surface stabilizers can preferably be selected from known organic and inorganic pharmaceutical excipients. Such excipients include various polymers, low molecular weight oligomers, natural products, and surfactants. Surface stabilizers include nonionic, cationic, zwitterionic, and ionic surfactants.

Representative examples of surface stabilizers include gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g.,* macrogol ethers such as cetomacrogol 1000), polyoxyethylene castor oil derivatives, polyoxyethylene sorbitan fatty acid esters (e.g., the commercially available Tweens^{®} such as *e.g.,* Tween 20^{®} and Tween 80^{®} (ICI Speciality Chemicals)); polyethylene glycols *(e.g.,* Carbowaxs 3550^{®} and 934^{®} (Union Carbide)), polyoxyethylene stearates, colloidal silicon dioxide, phosphates, sodium dodecylsulfate, carboxymethylcellulose calcium, carboxymethylcellulose sodium, methylcellulose, hydroxyethylcellulose, hydroxypropylcellulose, hydroxypropylmethyl-cellulose phthalate, noncrystalline cellulose, magnesium aluminium silicate, triethanolamine, polyvinyl alcohol (PVA), polyvinylpyrrolidone (PVP), 4-(1,1,3,3-tetramethylbutyl)-phenol polymer with ethylene oxide and formaldehyde (also known as tyloxapol, superione, and triton), poloxamers (*e.g.,* Pluronics F68^{®} and F108^{®}, which are block copolymers of ethylene oxide and propylene oxide); poloxamines (e.g., Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508) (BASF Wyandotte Corporation), dialkylesters of sodium sulfosuccinic acid (e.g., Aerosol OT^{®}, which is a dioctyl ester of sodium sulfosuccinic acid (DOSS) (American Cyanamid)); Duponol P^{®}, which is a sodium lauryl sulfate (DuPont); Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-IOG^{®} or Surfactant 10-G^{®} (Olin Chemicals, Stamford, CT); Crodestas SL-40^{®} (Croda, Inc.); and SA9OHCO, which is C₁₈H₃₇CH₂(CON(CH₃)-CH₂(CHOH)₄(CH₂0H)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336™), POLYQUAT 10™ (polyquaternium 10; Buckman Laboratories, TN), tetrabutylammonium bromide, benzyl trimethylammonium bromide, choline esters (such as choline esters of fatty acids), benzalkonium chloride, stearalkonium chloride compounds (such as stearyltrimonium chloride and Di-stearyldimonium chloride), cetyl pyridinium bromide or chloride, halide salts of quatemized polyoxyethylalkylamines, MIRAPOL^{™} (quaternized ammonium salt polymers) and ALKAQUAT^{™} (benzalkonium chloride) (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Nonpolymeric surface stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quatemium-5), dodecyl dimethyl ethylbenzyl ammonium chloride(Quatemium-14), Quatemium-22, Quaternium-26, Quatemium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

The surface stabilizers are commercially available and/or can be prepared by techniques known in the art. Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000), specifically incorporated by reference.

### 4. Nanoparticulate Angiogenesis Inhibitor/ Surface Stabilizer Particle Size

As used herein, particle size is determined on the basis of the weight average particle size as measured by conventional particle size measuring techniques well known to those skilled in the art. Such techniques include, for example, sedimentation field flow fractionation, photon correlation spectroscopy, light scattering, and disk centrifugation.

The nanoparticulate angiogenesis inhibitor compositions of the invention have an effective average particle size of less than about 2 microns. In preferred embodiments, the effective average particle size of the angiogenesis inhibitor particles is less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, or less than about 50 nm, when measured by the above techniques.

By "an effective average particle size of less than about 2000 nm" it is meant that at least 50% of the angiogenesis inhibitor particles have a particle size of less than about 2000 nm, by weight, when measured by the above techniques. Preferably, at least about 70%, about 90%, about 95%, or about 99% of the particles have a particle size of less than the effective average, *i.e.,* less than about 2000 nm, less than about 1900 nm, less than about 1800 nm, *etc..*

If the nanoparticulate angiogenesis inhibitor composition additionally comprises one or more non-angiogenesis inhibitor nanoparticulate active agents, then such active agents have an effective average particle size of less than about 2000 nm (*i.e.,* 2 microns), less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

If the nanoparticulate angiogenesis inhibitor is combined with a conventional or microparticulate angiogenesis inhibitor or non-angiogenesis inhibitor composition, then such a conventional composition is either solubilized or has an effective average particle size of greater than about 2 microns. By "an effective average particle size of greater than about 2 microns" it is meant that at least 50% of the conventional angiogenesis inhibitor or active agent particles have a particle size of greater than about 2 microns, by weight, when measured by the above-noted techniques. In other embodiments of the invention, at least about 70%, about 90%, about 95%, or about 99% of the conventional angiogenesis inhibitor or active agent particles have a particle size greater than about 2 microns.

### 5. Other Pharmaceutical Excipients

Pharmaceutical compositions according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents are lactose monohydrate, lactose anhydrous, and various starches; examples of binding agents are various celluloses and cross-linked polyvinylpyrrolidone, microcrystalline cellulose, such as Avicel® PH101 and Avicel® PH102, microcrystalline cellulose, and silicified microcrystalline cellulose (ProSolv SMCC™).

Suitable lubricants, including agents that act on the flowability of the powder to be compressed, are colloidal silicon dioxide, such as Aerosil^{®} 200, talc, stearic acid, magnesium stearate, calcium stearate, and silica gel.

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### 6. Concentration of Nanoparticulate Angiogenesis inhibitor and Stabilizer

The relative amount of angiogenesis inhibitor and one or more surface stabilizers can vary widely. The optimal amount of the surface stabilizers can depend, for example, upon the particular angiogenesis inhibitor selected, the hydrophilic lipophilic balance (HLB), melting point, water solubility of the surface stabilizer, and the surface tension of water solutions of the stabilizer, *etc.*

The concentration of the at least one angiogenesis inhibitor can vary from about 99.5% to about 0.001%, from about 95% to about 0.1%, or from about 90% to about 0.5%, by weight, based on the total combined weight of the at least one angiogenesis inhibitor and at least one surface stabilizer, not including other excipients.

The concentration of the one or more surface stabilizers can vary from about 0.5% to about 99.999%, from about 5.0% to about 99.9%, or from about 10% to about 99.5%, by weight, based on the total combined dry weight of the at least one angiogenesis inhibitor and at least one surface stabilizer, not including other excipients.

### E. Methods of Making Nanoparticulate Formulations

The nanoparticulate angiogenesis inhibitor compositions can be made using, for example, milling, precipitation, or homogenization techniques. Exemplary methods of making nanoparticulate compositions are described in the '684 patent. Methods of making nanoparticulate compositions are also described in U.S. Patent No. 5,518,187, for "Method of Grinding Pharmaceutical Substances;" U.S. Patent No. 5,718,388, for "Continuous Method of Grinding Pharmaceutical Substances;" U.S. Patent No. 5,862,999, for "Method of Grinding Pharmaceutical Substances;" U.S. Patent No. 5,665,331, for "Co-Microprecipitation of Nanoparticulate Pharmaceutical Agents with Crystal Growth Modifiers;" U.S. Patent No. 5,662,883, for "Co-Microprecipitation of Nanoparticulate Pharmaceutical Agents with Crystal Growth Modifiers;" U.S. Patent No. 5,560,932, for "Microprecipitation of Nanoparticulate Pharmaceutical Agents;" U.S. Patent No. 5,543,133, for "Process of Preparing X-Ray Contrast Compositions Containing Nanoparticles;" U.S. Patent No. 5,534,270, for "Method of Preparing Stable Drug Nanoparticles;" U.S. Patent No. 5,510,118, for "Process of Preparing Therapeutic Compositions Containing Nanoparticles;" and U.S. Patent No. 5,470,583, for "Method of Preparing Nanoparticle Compositions Containing Charged Phospholipids to Reduce Aggregation," all of which are specifically incorporated by reference.

One or more non-angiogenesis inhibitor active agents can be reduced in size at the same time as the angiogenesis inhibitor, to produce a nanoparticulate angiogenesis inhibitor and nanoparticulate non-angiogenesis inhibitor active agent composition. A non-angiogenesis inhibitor active agent, which is either conventional or nanoparticulate sized, can also be added to the nanoparticulate angiogenesis inhibitor composition after particle size reduction.

In yet another embodiment of the invention, nanoparticulate angiogenesis inhibitor compositions of the invention can be made in which the formulation comprises multiple nanoparticulate angiogenesis inhibitor compositions, each of which has a different effective average particle size. Such a composition can be made by preparing the individual nanoparticulate angiogenesis inhibitor compositions using, for example, milling, precipitation, or homogenization techniques, followed by combining the different compositions to prepare a single dosage form.

The nanoparticulate angiogenesis inhibitor compositions can be utilized in solid or liquid dosage formulations, such as liquid dispersions, gels, aerosols, ointments, creams, controlled release formulations, fast melt formulations, lyophilized formulations, tablets, capsules, delayed release formulations, extended release formulations, pulsatile release formulations, mixed immediate release and controlled release formulations, *etc.*

### 1. Milling to Obtain Nanoparticulate Dispersions

Milling of aqueous angiogenesis inhibitors to obtain a nanoparticulate dispersion comprises dispersing angiogenesis inhibitor particles in a liquid dispersion medium in which the angiogenesis inhibitor is poorly soluble, followed by applying mechanical means in the presence of grinding media to reduce the particle size of the angiogenesis inhibitor to the desired effective average particle size. The angiogenesis inhibitor particles can be reduced in size in the presence of at least one surface stabilizer. Alternatively, the angiogenesis inhibitor particles can be contacted with one or more surface stabilizers either before or after attrition. Other compounds, such as a diluent, can be added to the angiogenesis inhibitor/surface stabilizer composition either before, during, or after the size reduction process. Dispersions can be manufactured continuously or in a batch mode.

### 2. Precipitation to Obtain Nanoparticulate Angiogenesis Inhibitor Compositions

Another method of forming the desired nanoparticulate angiogenesis inhibitor composition is by microprecipitation. This is a method of preparing stable dispersions of angiogenesis inhibitors in the presence of one or more surface stabilizers and one or more colloid stability enhancing surface active agents free of any trace toxic solvents or solubilized heavy metal impurities. Such a method comprises, for example: (1) dissolving at least one angiogenesis inhibitor in a suitable solvent; (2) adding the formulation from step (1) to a solution comprising at least one surface stabilizer to form a clear solution; and (3) precipitating the formulation from step (2) using an appropriate non-solvent. The method can be followed by removal of any formed salt, if present, by dialysis or diafiltration and concentration of the dispersion by conventional means. Dispersions can be manufactured continuously or in a batch mode.

### 3. Homogenization to Obtain Nanoparticulate Angiogenesis Inhibitor Compositions

Exemplary homogenization methods of preparing nanoparticulate compositions are described in U.S. Patent No. 5,510,118, for "Process of Preparing Therapeutic Compositions Containing Nanoparticles." Such a method comprises dispersing angiogenesis inhibitor particles in a liquid dispersion medium, followed by subjecting the dispersion to homogenization to reduce the particle size of the angiogenesis inhibitor to the desired effective average particle size. The angiogenesis inhibitor particles can be reduced in size in the presence of at least one surface stabilizer. Alternatively, the angiogenesis inhibitor particles can be contacted with one or more surface stabilizers either before or after attrition. Other compounds, such as a diluent, can be added to the angiogenesis inhibitor/surface stabilizer composition either before, during, or after the size reduction process. Dispersions can be manufactured continuously or in a batch mode.

### F. Methods of Using Nanoparticulate Angiogenesis Inhibitor Formulations Comprising One or More Surface Stabilizers

The angiogenesis inhibitor compositions of the invention are useful in treating or preventing, for example, tumor growth, cancer growth, or any mammalian disease characterized by undesirable angiogenesis.

The nanoparticulate compositions of the present invention can be administered to humans and animals in any pharmaceutically acceptable manner, including, but not limited to orally, pulmonary, rectally, ocularly, colonicly, parenterally (e.g., intravenous, intramuscular, or subcutaneous), intracisternally, intravaginally, intraperitoneally, locally (e.g., powders, ointments, or drops), buccally, nasal, and topically. As used herein, the term "subject" is used to mean an animal, preferably a mammal, including a human or non-human. The terms patient and subject may be used interchangeably.

Compositions suitable for parenteral injection may comprise physiologically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents, or vehicles including water, ethanol, polyols (propylene glycol, polyethylene-glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

The nanoparticulate angiogenesis inhibitor compositions may also contain adjuvants such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

Solid dosage forms for oral administration include capsules, tablets, pills, powders, and granules. In such solid dosage forms, the nanoparticulate angiogenesis inhibitor is admixed with at least one of the following: (a) one or more inert excipients (or carrier), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to the angiogenesis inhibitor, the liquid dosage forms may comprise inert diluents commonly used in the art, such as water or other solvents, solubilizing agents, and emulsifiers. Besides such inert diluents, the composition can also include adjuvants, such as wetting agents, emulsifying and suspending agents, sweetening, flavoring, and perfuming agents.

One of ordinary skill will appreciate that effective amounts of an angiogenesis inhibitor can be determined empirically and can be employed in pure form or, where such forms exist, in pharmaceutically acceptable salt, ester, or prodrug form. Actual dosage levels of angiogenesis inhibitor in the nanoparticulate compositions of the invention may be varied to obtain an amount of active ingredient that is effective to obtain a desired therapeutic response for a particular composition and method of administration. The selected dosage level therefore depends upon the desired therapeutic effect, the route of administration, the potency of the angiogenesis inhibitor, the desired duration of treatment, and other factors.

The daily dose may be administered in single or multiple doses. It will be understood, however, that the specific dose level for any particular patient will depend upon a variety of factors including the body weight, general health, sex, diet, time and route of administration, potency of the administered angiogenesis inhibitor, rates of absorption and excretion, combination with other drugs and the severity of the particular disease being treated, and like factors well known in the medical arts..

The following example is given to illustrate the present invention. It should be understood, however, that the invention is not to be limited to the specific conditions or details described in this example. Throughout the specification, any and all references to a publicly available document, including a U.S. patent, are specifically incorporated by reference.

### Example 1

The purpose of this example was to describe how a nanoparticulate dispersion of an angiogenesis inhibitor can be made.

Nanocrystalline dispersions of an angiogenesis inhibitor can be made by milling the compound, at least one surface stabilizer, and any desired excipients on a suitable mill, such as a Netzsch Mill (Netzsch Inc., Exton, PA) or a Dyno-Mill, for a suitable time at a suitable temperature. 500 micron PolyMill media can be used.

### Example 2

The purpose of this example was to prepare a nanoparticulate composition of 2-methoxyestradiol, which is an angiogenesis inhibitor.

A nanoparticulate dispersion of 2-methoxyestradiol, having 5% (w/w) 2-methoxyestradiol, 1% (w/w) hydroxypropyl cellulose, low viscosity (HPC-SL), and 0.05% (w/w) docusate sodium (DOSS), was milled for 1 hour under high energy milling conditions in a NanoMill®-001 System (Custom Machine and Design Inc., Oxford, PA; *see* U.S. Patent No. 6,431,478 for "Small Scale Mill") equipped with a 10 cc chamber and utilizing 500 µm polymeric attrition media.

Following milling, the final mean particle size (volume statistics) of the nanoparticulate dispersion of 2-methoxyestradiol was 153 nm, with 50% < 144 nm, 90% < 217 nm, and 95% < 251 nm, measured using a Horiba LA-910 Laser Scattering Particle Size Distribution Analyzer (Horiba Instruments, Irvine, CA). Following two weeks storage at 5°C, the nanoparticulate dispersion of 2-methoxyestradiol had a mean particle size of 195 nm.

This example demonstrates the successful preparation of a stable nanoparticulate composition of an angiogenesis inhibitor. The angiogenesis inhibitor composition having a very small effective average particle size can be sterile filtered, which is particularly useful for injectable products, and for administration to immunocompromised patients, the elderly, and infants or juveniles.

### Example 3

The purpose of this example was to prepare a nanoparticulate composition of 2-methoxyestradiol.

A nanoparticulate dispersion of 2-methoxyestradiol, having 5% (w/w) 2-methoxyestradiol, 1% (w/w) hydroxypropyl methylcellulose (HPMC), and 0.05% (w/w) DOSS, was milled for 1 hour under high energy milling conditions in a NanoMill®-001 System (Custom Machine and Design Inc., Oxford, PA) equipped with a 10 cc chamber and utilizing 500 µm polymeric attrition media.

Following milling, the final mean particle size (volume statistics) of the nanoparticulate dispersion of 2-methoxyestradiol was 162 nm, with 50% < 151 nm, 90% < 234 nm, and 95% < 277 nm, measured using a Horiba LA-910 Laser Scattering Particle Size Distribution Analyzer (Horiba Instruments, Irvine, CA). Following two weeks storage at 5°C, the nanoparticulate dispersion of 2-methoxyestradiol had a mean particle size of 193 nm.

This example demonstrates the successful preparation of a stable nanoparticulate composition of an angiogenesis inhibitor.

### Example 4

The purpose of this example was to prepare a nanoparticulate composition of 2-methoxyestradiol.

A nanoparticulate dispersion of 2-methoxyestradiol, having 5% (w/w) 2-methoxyestradiol, 1% (w/w) HPC-SL, and 0.05% (w/w) DOSS, was milled for 1.5 hours under high energy milling conditions in a DYNO®-Mill KDL (Willy A. Bachofen AG, Maschinenfabrik, Basel, Switzerland) equipped with a 150 cc batch chamber and utilizing 500 µm polymeric attrition media.

The final mean particle size (volume statistics) of the nanoparticulate dispersion of 2-methoxyestradiol following milling was 157 nm, with 50% < 152 nm, 90% < 212 nm, and 95% < 236 nm, measured using a Horiba LA-910 Laser Scattering Particle Size Distribution Analyzer (Horiba Instruments, Irvine, CA). Following storage for one month at 5°C, 25°C, and 40°C, the nanoparticulate dispersion of 2-methoxyestradiol had a mean particle size of 207 nm, 216 nm, and 260 nm, respectively.

This example demonstrates the successful preparation of a stable nanoparticulate composition of an angiogenesis inhibitor.

### Example 5

The purpose of this example was to prepare a nanoparticulate composition of 2-methoxyestradiol.

A nanoparticulate dispersion of 2-methoxyestradiol, having 5% (w/w) 2-methoxyestradiol, 1% (w/w) HPMC, and 0.05% (w/w) DOSS, was milled for 2 hours under high energy milling conditions in a DYNO®-Mill KDL (Willy A. Bachofen AG, Maschinenfabrik, Basel, Switzerland) equipped with a 150 cc batch chamber and utilizing 500 µm polymeric attrition media.

The final mean particle size (volume statistics) of the nanoparticulate dispersion of 2-methoxyestradiol following milling was 157 nm, with 50% < 151 nm, 90% < 213 nm, and 95% < 240 nm, measured using a Horiba LA-910 Laser Scattering Particle Size Distribution Analyzer (Horiba Instruments, Irvine, CA). Following storage for one month at 5°C, 25°C, and 40°C, the nanoparticulate dispersion of 2-methoxyestradiol had a mean particle size of 182 nm, 198 nm, and 218 nm, respectively.

This example demonstrates the successful preparation of a stable nanoparticulate composition of an angiogenesis inhibitor.

### Example 6

The purpose of this example was to prepare a nanoparticulate composition of 2-methoxyestradiol.

A nanoparticulate dispersion of 2-methoxyestradiol, having 15% (w/w) 2-methoxyestradiol and 4% (w/w) lysozyme was milled for 1.5 hours under high energy milling conditions in a DYNO®-Mill KDL (Willy A. Bachofen AG, Maschinenfabrik, Basel, Switzerland) equipped with a 150 cc batch chamber and utilizing 500 µm polymeric attrition media.

The final mean particle size (volume statistics) of the nanoparticulate dispersion of 2-methoxyestradiol following milling was 110 nm, with 50% < 101 nm, 90% < 169 nm, and 95% < 204 nm, measured using a Horiba LA-910 Laser Scattering Particle Size Distribution Analyzer (Horiba Instruments, Irvine, CA). Following storage for one month at 5°C, 25°C, and 40°C, the nanoparticulate dispersion of 2-methoxyestradiol had a mean particle size of 190 nm, 201 nm, and 202 nm, respectively.

This example demonstrates the successful preparation of a stable nanoparticulate composition of an angiogenesis inhibitor.

### Example 7

The purpose of this example was to prepare a nanoparticulate composition of 2-methoxyestradiol.

A nanoparticulate dispersion of 2-methoxyestradiol, having 15% (w/w) 2-methoxyestradiol, 3% (w/w) copovidonum, and 0.15% (w/w) DOSS, was milled for 1.5 hours under high energy milling conditions in a DYNO®-Mill KDL (Willy A. Bachofen AG, Maschinenfabrik, Basel, Switzerland) equipped with a 150 cc batch chamber and utilizing 500 µm polymeric attrition media.

The final mean particle size (volume statistics) of the nanoparticulate dispersion of 2-methoxyestradiol following milling was 155 nm, with 50% < 148 nm, 90% < 217 nm, and 95% < 245 nm, measured using a Horiba LA-910 Laser Scattering Particle Size Distribution Analyzer (Horiba Instruments, Irvine, CA). Following storage for 1.5 months at 5°C and 25°C, the nanoparticulate dispersion of 2-methoxyestradiol had a mean particle size of 209 nm and 216 nm, respectively.

This example demonstrates the successful preparation of a stable nanoparticulate composition of an angiogenesis inhibitor.

### Example 8

The purpose of this example was to prepare a nanoparticulate composition of 2-methoxyestradiol.

A nanoparticulate dispersion of 2-methoxyestradiol, having 25% (w/w) 2-methoxyestradiol, 5% (w/w) HPMC, and 0.25% (w/w) DOSS, was milled for 12.6 hours under high energy milling conditions in a NanoMill®-02 System utilizing 500 µm polymeric attrition media.

The final mean particle size (volume statistics) of the nanoparticulate dispersion of 2-methoxyestradiol following milling was 149 nm, with 50% < 145 nm, 90% < 203 nm, and 95% < 223 nm, measured using a Horiba LA-910 Laser Scattering Particle Size Distribution Analyzer (Horiba Instruments, Irvine, CA). Following storage for one month at 5°C, 25°C, and 40°C, the nanoparticulate dispersion of 2-methoxyestradiol had a mean particle size of 163 nm, 164 nm, and 167 nm, respectively.

This example demonstrates the successful preparation of a stable nanoparticulate composition of an angiogenesis inhibitor.

### Example 9

The purpose of this example was to prepare a nanoparticulate composition of 2-methoxyestradiol.

A nanoparticulate dispersion of 2-methoxyestradiol, having 25% (w/w) 2-methoxyestradiol, 5% (w/w) HPMC, and 0.05% (w/w) DOSS, was milled for 3.5 hours under high energy milling conditions in a DYNO®-Mill KDL (Willy A. Bachofen AG, Maschinenfabrik, Basel, Switzerland) equipped with a 600 cc recirculation chamber and utilizing 500 µm polymeric attrition media.

The final mean particle size (volume statistics) of the nanoparticulate dispersion of 2-methoxyestradiol following milling was 146 nm, with 50% < 143 nm, 90% < 194 nm, and 95% < 215 nm, measured using a Horiba LA-910 Laser Scattering Particle Size Distribution Analyzer (Horiba Instruments, Irvine, CA). The sample showed aggregation after 4 days at 5°C and had a mean particle size of 1968 nm.

This example demonstrates that not all combinations of angiogenesis inhibitors and surface stabilizers, at all concentrations, will result in a stable nanoparticulate composition of an angiogenesis inhibitor.

### Example 10

The purpose of this example was to prepare a nanoparticulate composition of 2-methoxyestradiol.

A nanoparticulate dispersion of 2-methoxyestradiol, having 25% (w/w) 2-methoxyestradiol, 5% (w/w) HPMC, and 0.25% (w/w) DOSS, was milled for 5.5 hours under high energy milling conditions in a DYNO®-Mill KDL (Willy A. Bachofen AG, Maschinenfabrik, Basel, Switzerland) equipped with a 600 cc recirculation chamber and utilizing 500 µm polymeric attrition media.

The final mean particle size (volume statistics) of the nanoparticulate dispersion of 2-methoxyestradiol following milling was 148 nm, with 50% < 144 nm, 90% < 201 nm, and 95% < 221 nm, measured using a Horiba LA-910 Laser Scattering Particle Size Distribution Analyzer (Horiba Instruments, Irvine, CA). Following storage for 4 months at 5°C, 25°C, and 40°C, the nanoparticulate dispersion of 2-methoxyestradiol had a mean particle size of 186 nm, 229 nm, and 220 nm, respectively.

This example demonstrates the successful preparation of a stable nanoparticulate composition of an angiogenesis inhibitor.

It will be apparent to those skilled in the art that various modifications and variations can be made in the methods and compositions of the present invention without departing from the spirit or scope of the invention. Thus, it is intended that the present invention cover the modifications and variations of this invention provided they come within the scope of the appended claims and their equivalents.

The following pages 41 to 64 contain preferred embodiments. Accordingly, the term "claim" as used therein refers to such a "preferred embodiment".

## Claims

1. A nanoparticulate angiogenesis inhibitor composition comprising:
(a) particles of an angiogenesis inhibitor or a salt thereof having an effective average particle size of less than about 2000 nm; and
(b) associated with the surface thereof at least one surface stabilizer.

2. The composition of claim 1, wherein the angiogenesis inhibitor is selected from the group consisting of medroxyprogesterone acetate, 2-methoxyestradiol, prinomastat, batimastat, BAY 12-9566, carboxyamidotriazole, CC-1088, dextromethorphan acetic, dimethylxanthenone acetic acid, EMD 121974, endostatin, IM-862, marimastat, matrix metalloproteinase, penicillamine, PTK787/ZK 222584, RPI.4610, squalamine, squalamine lactate, SU5416, (±)-thalidomide, S-thalidomide, R- thalidomide, TNP-470, combretastatin, paclitaxel, tamoxifen, COL-3, neovastat, BMS-275291, SU6668, interferon-alpha, anti-VEGF antibody, Medi-522 (Vitaxin II), CAI, celecoxib, Interleukin-12, IM862, Amilloride, Angiostatin Protein, Angiostatin K1-3, Angiostatin K1-5, Captopril, DL-alpha-Difluoromethylornithine, DL-alpha-Difluoromethylornithine HCl, His-Tag Endostatin Protein, Fumagillin, Herbimycin A, 4-Hydroxyphenylretinamide, gamma-interferon, Juglone, Laminin, Laminin Hexapeptide, Laminin Pentapeptide, Lavendustin A, Medroxyprogesterone, Minocycline, Minocycline HCl, Placental Ribonuclease Inhibitor, Suramin, Sodium Salt Suramin, Human Platelet Thrombospondin, Tissue Inhibitor of Metalloproteinase 1, Neutrophil Granulocyte Tissue Inhibitor of Metalloproteinase 1, and Rheumatoid Synovial Fibroblast Tissue Inhibitor of Metalloproteinase 2.

3. The composition of claim 1 or claim 2, wherein the angiogenesis inhibitor is 2-methoxyestradiol.

4. The composition of any one of claims 1-3, wherein the angiogenesis inhibitor is selected from the group consisting of a crystalline phase, an amorphous phase, a semi-crystalline phase, a semi-amorphous phase, and mixtures thereof.

5. The composition of any one of claims 1-4, wherein the effective average particle size of the nanoparticulate angiogenesis inhibitor is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.

6. The composition of any one of claims 1-5, wherein the composition is formulated for administration selected from the group consisting of oral, pulmonary, rectal, opthalmic, colonic, parenteral, intracisternal, intravaginal, intraperitoneal, local, buccal, nasal, and topical administration, or wherein the composition is formulated into a dosage form selected from the group consisting of liquid dispersions, gels, aerosols, ointments, creams, controlled release formulations, fast melt formulations, lyophilized formulations, tablets, capsules, delayed release formulations, extended release formulations, pulsatile release formulations, and mixed immediate release and controlled release formulations.

7. The composition of any one of claims 1-6, wherein the composition further comprises one or more pharmaceutically acceptable excipients, carriers, or a combination thereof.

8. The composition of any one of claims 1-7, wherein the angiogenesis inhibitor is present in an amount selected from the group consisting of from about 99.5% to about 0.001%, from about 95% to about 0.1%, and from about 90% to about 0.5%, by weight, based on the total combined weight of the angiogenesis inhibitor and at least one surface stabilizer, not including other excipients.

9. The composition of any one of claims 1-8, wherein the at least one surface stabilizer is present in an amount selected from the group consisting of from about 0.5% to about 99.999%, from about 5.0% to about 99.9%, and from about 10% to about 99.5%, by weight, based on the total combined weight of the at least one angiogenesis inhibitor and at least one surface stabilizer, not including other excipients.

10. The composition of any one of claims 1-9, comprising at least two surface stabilizers.

11. The composition of any one of claims 1-10, wherein the surface stabilizer is selected from the group consisting of an anionic surface stabilizer, a cationic surface stabilizer, an ionic surface stabilizer, and a zwitterionic surface stabilizer.

12. The composition of any one of claims 1-11, wherein at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, C₁₈H₃₇CH₂C(O)N(CH₃)-CH₂(CHOH)4(CH₂OH)₂, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, PEG-vitamin E, and random copolymers of vinyl acetate and vinyl pyrrolidone.

13. The composition of any one of claims 1-11, wherein the surface stabilizer is selected from the group consisting of cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL^{™}, ALKAQUAT^{™}, alkyl pyridinium salts; amines, amine salts, amine oxides, imide azolinium salts, protonated quaternary acrylamides, methylated quaternary polymers, and cationic guar, particularly wherein the composition is bioadhesive.

14. The composition of any one of claims 1-13, wherein the composition comprises more than one angiogenesis inhibitor, particularly wherein at least one angiogenesis inhibitor has an effective average particle size which is greater than about 2 microns.

15. The composition of any one of claims 1-14, additionally comprising at least one nanoparticulate angiogenesis inhibitor composition having an effective average particle size of less than about 2 microns, wherein said additional nanoparticulate angiogenesis inhibitor composition has an effective average particle size which is different than the particle size of the nanoparticulate angiogenesis inhibitor composition of claim 1.

16. The composition of any one of claims 1-15, additionally comprising at least one non-angiogenesis inhibitor active agent, particularly wherein said active agent is selected from the group consisting of amino acids, proteins, peptides, nucleotides, anti-obesity drugs, nutraceuticals, dietary supplements, central nervous symptom stimulants, carotenoids, corticosteroids, elastase inhibitors, anti-fungals, alkylxanthine, oncology therapies, anti-emetics, analgesics, opioids, antipyretics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, alpha-adrenergic receptor blocking agents, beta-adrenoceptor blocking agents, blood products, blood substitutes, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio- pharmaceuticals, sex hormones, anti-allergic agents, stimulants, anoretics, sympathomimetics, thyroid agents, vasodilators, vasomodulator, xanthines, Mu receptor antagonists, Kappa receptor antagonists, non-narcotic analgesics, monoamine uptake inhibitors, adenosine regulating agents, cannabinoid derivatives, Substance P antagonists, neurokinin-1 receptor antagonists, and sodium channel blockers, more particularly wherein said nutraceutical is selected from the group consisting of lutein, folic acid, fatty acids, fruit extracts, vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish oils, marine animal oils, and probiotics.

17. The composition of claim 16, wherein at least one non-angiogenesis inhibitor active agent has an effective average particle size of less than about 2 microns, or wherein at least one non-angiogenesis inhibitor active agent has an effective average particle size of greater than about 2 microns.

18. The composition of any one of claims 1-17, wherein upon administration the composition redisperses such that the angiogenesis inhibitor particles have a particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, and less than about 50 nm.

19. The composition of any one of claims 1-18, wherein the composition does not produce significantly different absorption levels when administered under fed as compared to fasting conditions.

20. The composition of any one of claims 1-19, wherein the difference in absorption of the nanoparticulate angiogenesis inhibitor composition of the invention, when administered in the fed versus the fasted state, is selected from the group consisting of less than about 100%, less than about 90%, less than about 80%, less than about 70%, less than about 60%, less than about 50%, less than about 40%, less than about 35%, less than about 30%, less than about 25%, less than about 20%, less than about 15%, less than about 10%, less than about 5%, and less than about 3%.

21. The composition of any one of claims 1-20, wherein the composition does not produce significantly different rates of absorption (Tₘₐₓ) when administered under fed as compared to fasting conditions.

22. The composition of any one of claims 1-21, wherein the difference in the Tₘₐₓ for the nanoparticulate angiogenesis inhibitor composition of the invention, when administered in the fed versus the fasted state, is less than about 100%, less than about 90%, less than about 80%, less than about 70%, less than about 60%, less than about 50%, less than about 40%, less than about 30%, less than about 20%, less than about 15%, less than about 10%, less than about 5%, and less than about 3%.

23. The composition of any one of claims 1-22, wherein upon administration the Tₘₐₓ is less than that of a conventional non-nanoparticulate composition of the same angiogenesis inhibitor, administered at the same dosage.

24. The composition of any one of claims 1-23, wherein in comparative pharmacokinetic testing with a non-nanoparticulate composition of the same angiogenesis inhibitor, administered at the same dosage, the nanoparticulate composition exhibits a Tₘₐₓ selected from the group consisting of less than about 100%, less than about 90%, less than about 80%, less than about 70%, less than about 60%, less than about 50%, less than about 40%, less than about 30%, less than about 25%, less than about 20%, less than about 15%, and less than about 10% of the Tₘₐₓ exhibited by the non-nanoparticulate composition of the angiogenesis inhibitor.

25. The composition of any one of claims 1-24, wherein following administration the composition has a Tₘₐₓ selected from the group consisting of less than about 2.5 hours, less than about 2.25 hours, less than about 2 hours, less than about 1.75 hours, less than about 1.5 hours, less than about 1.25 hours, less than about 1.0 hours, less than about 50 minutes, less than about 40 minutes, less than about 30 minutes, less than about 25 minutes, less than about 20 minutes, less than about 15 minutes, and less than about 10 minutes.

26. The composition of any one of claims 1-25, wherein upon administration the Cₘₐₓ of the composition is greater than the Cₘₐₓ of a conventional non-nanoparticulate composition of the same angiogenesis inhibitor, administered at the same dosage.

27. The composition of any one of claims 1-26, wherein in comparative pharmacokinetic testing with a non-nanoparticulate composition of the same angiogenesis inhibitor, administered at the same dosage, the nanoparticulate composition exhibits a Cₘₐₓ selected from the group consisting of greater than about 5%, greater than about 10%, greater than about 15%, greater than about 20%, greater than about 30%, greater than about 40%, greater than about 50%, greater than about 60%, greater than about 70%, greater than about 80%, greater than about 90%, greater than about 100%, greater than about 110%, greater than about 120%, greater than about 130%, greater than about 140%, and greater than about 150% than the Cₘₐₓ exhibited by the non-nanoparticulate composition of the angiogenesis inhibitor.

28. A method of making an angiogenesis inhibitor composition comprising contacting particles of at least one angiogenesis inhibitor with at least one surface stabilizer for a time and under conditions sufficient to provide an angiogenesis inhibitor composition having an effective average particle size of less than about 2 microns.

29. The method of claim 28, wherein said contacting comprises grinding, particularly
wherein said grinding comprises wet grinding, or
wherein said contacting comprises homogenizing.

30. The method of claim 28, wherein said contacting comprises:
(a) dissolving the angiogenesis inhibitor particles in a solvent;
(b) adding the resulting angiogenesis inhibitor solution to a solution comprising at least one surface stabilizer; and
(c) precipitating the solubilized angiogenesis inhibitor having at least one surface stabilizer associated with the surface thereof by the addition thereto of a non-solvent.

31. The method of any one of claims 28-30, wherein the composition is defined as in claims 2, 4 and/or 5.

32. The method of any one of claims 28-31, wherein the angiogenesis inhibitor is present in an amount selected from the group consisting of from about 99% to about 0.001%, from about 95% to about 0.5%, and from about 90% to about 0.5%, by weight, based on the total combined weight of the angiogenesis inhibitor and at least one surface stabilizer, not including other excipients.

33. The method of any one of claims 28-32, wherein at least one surface stabilizer is present in an amount selected from the group consisting of from about 0.5% to about 99.999%, from about 5.0% to about 99.9%, and from about 10% to about 99.5%, by weight, based on the total combined dry weight of the angiogenesis inhibitor and at least one surface stabilizer, not including other excipients.

34. The method of any one of claims 28-33, wherein the angiogenesis inhibitor particles are contacted with at least two surface stabilizers.

35. The method of any one of claims 28-34, wherein the surface stabilizer is selected from the group consisting of an anionic surface stabilizer, a cationic surface stabilizer, an ionic surface stabilizer, and a zwitterionic surface stabilizer.

36. The method of any one of claims 28-35, wherein at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, C₁₈H₃₇CH₂C(O)N(CH₃)-CH₂(CHOH)₄(CH₂OH)₂, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, PEG-vitamin E, and random copolymers of vinyl acetate and vinyl pyrrolidone.

37. The method of any one of claims 28-35, wherein the surface stabilizer is selected from the group consisting of cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL^{™}, ALKAQUAT^{™}, alkyl pyridinium salts; amines, amine salts, amine oxides, imide azolinium salts, protonated quaternary acrylamides, methylated quaternary polymers, and cationic guar.

38. The method of any one of claims 28-37, wherein after preparation of a first nanoparticulate angiogenesis inhibitor composition, a second angiogenesis inhibitor composition having an effective average particle size of greater than about 2 microns is combined with the first angiogenesis inhibitor composition.

39. The method of any one of claims 28-38, wherein either prior or subsequent to preparation of the nanoparticulate angiogenesis inhibitor composition, at least one non-angiogenesis inhibitor active agent is added to the angiogenesis inhibitor composition, particularly
wherein said non-angiogenesis inhibitor active agent is selected from the group consisting of amino acids, proteins, peptides, nucleotides, anti-obesity drugs, nutraceuticals, dietary supplements, carotenoids, central nervous system stimulants, corticosteroids, elastase inhibitors, anti-fungals, alkylxanthine, oncology therapies, anti-emetics, analgesics, opioids, antipyretics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, alpha-adrenergic receptor blocking agents, beta-adrenoceptor blocking agents, blood products, blood substitutes, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio- pharmaceuticals, sex hormones, anti-allergic agents, stimulants, anoretics, sympathomimetics, thyroid agents, vasodilators, vasomodulator, xanthines, Mu receptor antagonists, Kappa receptor antagonists, non-narcotic analgesics, monoamine uptake inhibitors, adenosine regulating agents, cannabinoid derivatives, Substance P antagonists, neurokinin-1 receptor antagonists, and sodium channel blockers, more particularly
wherein said nutraceutical is selected from the group consisting of lutein, folic acid, fatty acids, fruit extracts, vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish oils, marine animal oils, and probiotics.

40. The method of claim 39, wherein at least one non-angiogenesis inhibitor active agent has an effective average particle size of less than about 2 microns, or
wherein at least one non- angiogenesis inhibitor active agent has an effective average particle size of greater than about 2 microns.

41. An angiogenesis inhibitor composition for use in a method of treating a subject, the method comprising administering to the subject an effective amount of an angiogenesis inhibitor composition comprising:
(a) particles of an angiogenesis inhibitor or a salt thereof having an effective average particle size of less than about 2000 nm; and
(b) associated with the surface thereof at least one surface stabilizer.

42. The composition of claim 41, wherein the composition is defined as in claims 2 and/or 4-13.

43. The composition of claim 41 or claim 42, wherein the surface stabilizer is selected from the group consisting of benzalkonium chloride, polymethylmethacrylate trimethylammonium bromide, polyvinylpyrrolidone-2-dimethylaminoethyl methacrylate dimethyl sulfate, hexadecyltrimethyl ammonium bromide, cationic lipids, sulfonium compounds, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL^{™}, ALKAQUAT^{™}, alkyl pyridinium salts; amines, amine salts, amine oxides, imide azolinium salts, protonated quaternary acrylamides, methylated quaternary polymers, and cationic guar.

44. The composition of any one of claims 41-43, comprising additionally administering a second angiogenesis inhibitor composition having an effective average particle size of greater than about 2 microns.

45. The composition of any one of claims 41-44, comprising additionally administering at least one non-angiogenesis inhibitor active agent, particularly
wherein said non-angiogenesis inhibitor active agent is selected from the group consisting of amino acids proteins, peptides, nucleotides, anti-obesity drugs, nutraceuticals, dietary supplements, carotenoids, central nervous system stimulants, corticosteroids, elastase inhibitors, anti-fungals, alkylxanthine, oncology therapies, anti-emetics, analgesics, opioids, antipyretics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, alpha-adrenergic receptor blocking agents, beta-adrenoceptor blocking agents, blood products, blood substitutes, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio- pharmaceuticals, sex hormones, anti-allergic agents, stimulants, anoretics, sympathomimetics, thyroid agents, vasodilators, vasomodulator, xanthines, Mu receptor antagonists, Kappa receptor antagonists, non-narcotic analgesics, monoamine uptake inhibitors, adenosine regulating agents, cannabinoid derivatives, Substance P antagonists, neurokinin-1 receptor antagonists, and sodium channel blockers more particularly
wherein said nutraceutical is selected from the group consisting of lutein, folic acid, fatty acids, fruit extracts, vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish oils, marine animal oils, and probiotics.

46. The composition of claim 45, wherein at least one non-angiogenesis inhibitor active agent has an effective average particle size of less than about 2 microns, or
wherein at least one non- angiogenesis inhibitor active agent has an effective average particle size of greater than about 2 microns.

47. The composition of any one of claims 41-46, wherein the composition does not produce significantly different absorption levels when administered under fed as compared to fasting conditions.

48. The composition of any one of claims 41-47, wherein the difference in absorption of the nanoparticulate angiogenesis inhibitor composition of the invention, when administered in the fed versus the fasted state, is selected from the group consisting of less than about 100%, less than about 90%, less than about 80%, less than about 70%, less than about 60%, less than about 50%, less than about 40%, less than about 35%, less than about 30%, less than about 25%, less than about 20%, less than about 15%, less than about 10%, less than about 5%, and less than about 3%.

49. The method of any one of claims 41-48, wherein the composition does not produce significantly different rates of absorption (Tₘₐₓ) when administered under fed as compared to fasting conditions.

50. The composition of any one of claims 41-49, wherein the difference in the Tₘₐₓ for the nanoparticulate angiogenesis inhibitor composition of the invention, when administered in the fed versus the fasted state, is less than about 100%, less than about 90%, less than about 80%, less than about 70%, less than about 60%, less than about 50%, less than about 40%, less than about 30%, less than about 20%, less than about 15%, less than about 10%, less than about 5%, and less than about 3%.

51. The composition of any one of claims 41-50, wherein upon administration the Tₘₐₓ is less than that of a conventional non-nanoparticulate composition of the same angiogenesis inhibitor, administered at the same dosage.

52. The composition of any one of claims 41-51, wherein the nanoparticulate angiogenesis inhibitor composition exhibits a Tₘₐₓ, as compared to a non-nanoparticulate composition of the same angiogenesis inhibitor administered at the same dosage, selected from the group consisting of less than about 90%, less than about 80%, less than about 70%, less than about 60%, less than about 50%, less than about 40%, less than about 30%, less than about 25%, less than about 20%, less than about 15%, and less than about 10% of the Tₘₐₓ exhibited by the non-nanoparticulate composition of the angiogenesis inhibitor.

53. The composition of any one of claims 41-52, wherein upon administration the Tₘₐₓ of the composition is selected from the group consisting of less than about 2.5 hours, less than about 2.25 hours, less than about 2 hours, less than about 1.75 hours, less than about 1.5 hours, less than about 1.25 hours, less than about 1.0 hours, less than about 50 minutes, less than about 40 minutes, less than about 30 minutes, less than about 25 minutes, less than about 20 minutes, less than about 15 minutes, and less than about 10 minutes.

54. The composition of any one of claims 41-53, wherein upon administration the Cₘₐₓ of the composition is greater than the Cₘₐₓ of a conventional non-nanoparticulate composition of the same angiogenesis inhibitor, administered at the same dosage.

55. The composition of any one of claims 41-54, wherein the nanoparticulate angiogenesis inhibitor composition exhibits a Cₘₐₓ, as compared to a non-nanoparticulate composition of the same angiogenesis inhibitor administered at the same dosage, selected from the group consisting of greater than about 5%, greater than about 10%, greater than about 15%, greater than about 20%, greater than about 30%, greater than about 40%, greater than about 50%, greater than about 60%, greater than about 70%, greater than about 80%, greater than about 90%, greater than about 100%, greater than about 110%, greater than about 120%, greater than about 130%, greater than about 140%, and greater than about 150% than the Cₘₐₓ exhibited by the non-nanoparticulate composition of the angiogenesis inhibitor.

56. The composition of any one of claims 41-55, wherein the subject is a human, and/or the method is used to treat an condition where a selective angiogenesis inhibitor is indicated, and/or
wherein the method is used to treat a mammalian disease **characterized by** undesirable angiogenesis and/or
wherein the method is used to treat or prevent tumor growth, and/or
wherein the method is used to treat or prevent cancer growth.

57. Use of a composition according to any of claims 41-56 for the manufacture of a medicament for the treatment of a condition where a selective angiogenesis inhibitor is indicated and/or a disease **characterized by** undesirable angiogenesis and/or for the treatment or prevention of tumor and/or cancer growth.

58. The composition of claim 41, wherein the angiogenesis inhibitor is medroxyprogesterone acetate.

59. Any product, method or use or any other subject-matter disclosed in the description including the examples.
